# EUROPEAN PATENT SPECIFICATION

(11) **EP 1 078 651 B1**
(45) Date of publication and mention of the grant of the patent: **07.05.2008**
(21) Application number: 00307349.1
(22) Date of filing: 25.08.2000
(51) Int. Cl.: A61N 1/368, A61N 1/362

(54) **Implantable pacemaker and method of detecting a pacemaker mediated tachycardia**
Implantierbarer Herzschrittmacher und Verfahren zur Detektion von durch einen Herzschrittmacher induzierten Tachykardien
Stimulateur cardiaque implantable et méthode pour détecter une tachycardie induite par un stimulateur

(30) Priority: 27.08.1999 US 384319
(43) Date of publication of application: 28.02.2001
(73) Proprietor: PACESETTER, INC., Sylmar, CA 91392-9221 (US)
(72) Inventor: Sloman, Laurence S., West Hollywood, CA 90069 (US); Mann, Brian M., Beverly Hills, CA 90210 (US)
(74) Representative: Rees, David Christopher

(56) References cited:
- US-A- 5 282 465
- US-A- 5 441 523
- US-A- 5 609 610

## Description

The present invention generally relates to an implantable pacemaker having pacemaker-mediated tachycardia (PMT) detection. The present invention more particularly relates to an implantable pacemaker having a Wenckebach upper rate-response with PMT detection at the maximum tracking rate.

Implantable pacemakers are well known in the art. A pacemaker is a medical device that is used to selectively stimulate the heart with electrical stimulation pulses to assist the heart in performing its pumping function. Normally, the stimulation pulses are timed to keep the heart rate above a prescribed limit, such as, for example, to treat a bradycardia.

A pacemaker may be considered as a pacing system. The pacing system is comprised of two major components. One component is a pulse generator which generates the stimulation pulses and includes the electronic circuitry and the power cell or battery. The other is the lead, or leads, which electrically couple the pacemaker to the heart.

The pacemaker delivers an electrical stimulus to the heart to cause the heart to contract when the patient's own intrinsic rhythm fails. To this end, pacemakers include sensing circuits that sense the heart electrogram, and in particular that sense the P-waves and/or R-waves in the electrogram. By monitoring such P-waves and/or R-waves, the pacemaker circuits are able to determine the intrinsic rhythm of the heart, and provide stimulation pulses that force atrial and/or ventricular depolarization at appropriate times in the cardiac cycle so as to help stabilize the electrical rhythm of the heart.

Pacemakers are described as either single-chamber or dual-chamber systems. A single-chamber system stimulates and senses the same chamber of the heart (atrium or ventricle). A dual-chamber system stimulates and/or senses in both chambers of the heart (atrium and ventricle). Dual-chamber systems may typically be programmed to operate in either a dual-chamber mode or a single-chamber mode.

A popular mode of operation for dual-chamber pacemakers is the DDD mode. Specifically, DDD systems provide atrial pacing during atrial bradycardia, ventricular pacing during ventricular bradycardia, and atrial and ventricular pacing during combined atrial and ventricular bradycardia. In addition, DDD systems provide an atrial synchronous mode. Such features more closely approximate the normal response to exercise, or other physiological activity demanding a faster heart rate, by permitting a rate increase to occur commensurate with the rate of the sensed P-waves. This advantageously increases cardiac output and facilitates maintenance of AV synchrony.

Most implantable pacemakers also include some type of upper rate limiting feature to prevent the pacemaker from providing stimulation pulses at a rate that exceeds a prescribed upper rate limit referred to as a maximum tracking rate. The response to upper rate limiting is commonly referred to as a Wenckebach response. The Wenckebach response occurs when the spontaneous intrinsic atrial rate rises above the preset or programmed upper rate limit of the pacemaker. The ventricular output pulses are delivered at the upper rate limit, which causes an effective prolongation of the AV delay. An intermittent block occurs when an intrinsic atrial event falls within the atrial refractory period of the pacer. The P-wave is not sensed by the pacer and, therefore, a ventricular output pulse is not synchronized to this atrial event. Hence, when the Wenckebach response occurs, some cardiac cycles will be elongated resulting in an unstable cardiac rate.

Another condition that may occur during atrial synchronous pacing is a pacemaker mediated tachycardia (PMT). A PMT results when the atrial sensing circuit detects a P-wave induced by a retrograde conducted ventricular pacing pulse response outside of the sensing circuits refractory period. When this occurs, the pacemaker subsequently initiates a paced ventricular beat. Repeated stimulation is sustained by heart tissue retrograde conduction and by pacemaker antegrade conduction.

Methods for preventing PMT are well known in the art. One such known method involves the use of programmable atrial refractory periods, where the atrial refractory period is programmed to be longer than the retrograde conduction interval after a predetermined number of cardiac cycles, for example, ten cardiac cycles, at the maximum rate. Another known method is based on the fact that the majority of PMTs are initiated by ventricular premature beats wherein a ventricular event is not preceded by an atrial beat. Thus, in this method, a ventricular premature beat causes a prolonged atrial refractory period. Still another method is to trigger a simultaneous atrial stimulation with a ventricular premature beat causing the atrium to be refractory when the retrograde conduction occurs.

Most dual-chamber pacemakers that include a PMT breaking algorithm or therapy also include a means for restricting the application of the PMT breaking therapy. For example, it has been proposed that the PV delay be varied in order to check for a constant VP interval. While this is necessary for PMTs that occur below the maximum tracking rate, alternate criteria may be used at maximum tracking rate for pacemakers with a Wenckebach upper rate-response. In accordance with the present invention, a system and method provides such an alternate approach for detecting a PMT at the maximum tracking rate for pacemakers with a Wenckebach upper rate-response.

US-A-5,282,465 discloses a dual chamber pacemaker which provides a Wenckebach response for optimising tracking of atrial signals having rates within a rate range above the normal tracking range. US-A-5,609,610 discloses a dual chamber pacemaker with automatic adaptation for intrinsic atrial events, in which the PVARP and alert interval are adaptably adjusted to optimise the PVARP of a patient without affecting the atrial-to-atrial interval.

The invention provides a system for detecting a pacemaker mediated tachycardia at a maximum tracking rate for use in an implantable pacemaker having a Wenckebach upper rate-response. As the Wenckebach response at the pacemaker maximum tracking rate results in an unstable cardiac rate, in accordance with the present invention, a PMT at the maximum tracking rate is detected when the cardiac rate is at the maximum tracking rate and is stable over a predetermined number of cardiac cycles. The features of the preamble of claim 1 are known from the disclosures US-A-5,282,465

More specifically, the PMT detection system includes a sensing circuit that senses cardiac events in an atrium of a heart. A pulse generator circuit delivers ventricular pacing pulses to a ventricle of the heart during a number of cardiac cycles sufficient to evoke a Wenckebach response. Each ventricular pacing pulse is delivered following a sensed atrial event. As the ventricular pacing pulses are delivered, the cardiac rate is determined for each cardiac cycle. After the number of cardiac cycles, it is determined if the cardiac rate is substantially constant during the number of cardiac cycles. A substantially constant cardiac rate during the number of cardiac cycles is indicative of a pacemaker mediated tachycardia at the maximum tracking rate.

The PMT detection system is preferably implemented by a microprocessor of the implantable pacemaker. The microprocessor controls the delivery of the ventricular pacing pulses, determines the cardiac rate during the number of cardiac cycles, and determines if the cardiac rate is substantially constant.

The PMT detection system of the present invention permits a pacemaker having a Wenckebach upper rate-response to respond quickly to pacemaker mediated tachycardias. In addition, the PMT detection system accurately determines the existence of retrograde conduction and reduces pauses in pacemaker therapy by reducing the occurrence of false PMT detections.

The above and other aspects, features, and advantages of the present invention will be more apparent from the following more particular description thereof, presented in conjunction with the following drawings wherein:
**FIG. 1** is a functional block diagram of an implantable dual-chamber pacemaker embodying the present invention;
**FIG. 2** is a timing diagram illustrating various heart and pacer events which occur during a Wenckebach upper rate-response;
**FIG. 3** is a timing diagram illustrating various heart and pacer events which occur during a PMT at the maximum rate;
**FIG. 4** shows a flowchart that illustrates PMT detection in accordance with a preferred embodiment of the present invention; and
**FIG. 5** shows another flowchart that illustrates, in greater detail, PMT detection in accordance with the present invention.

The following description is of the best mode presently contemplated for carrying out the invention. This description is not to be taken in a limiting sense, but is made merely for the purpose of describing the general principles of the invention. The scope of the invention should be determined with reference to the claims.

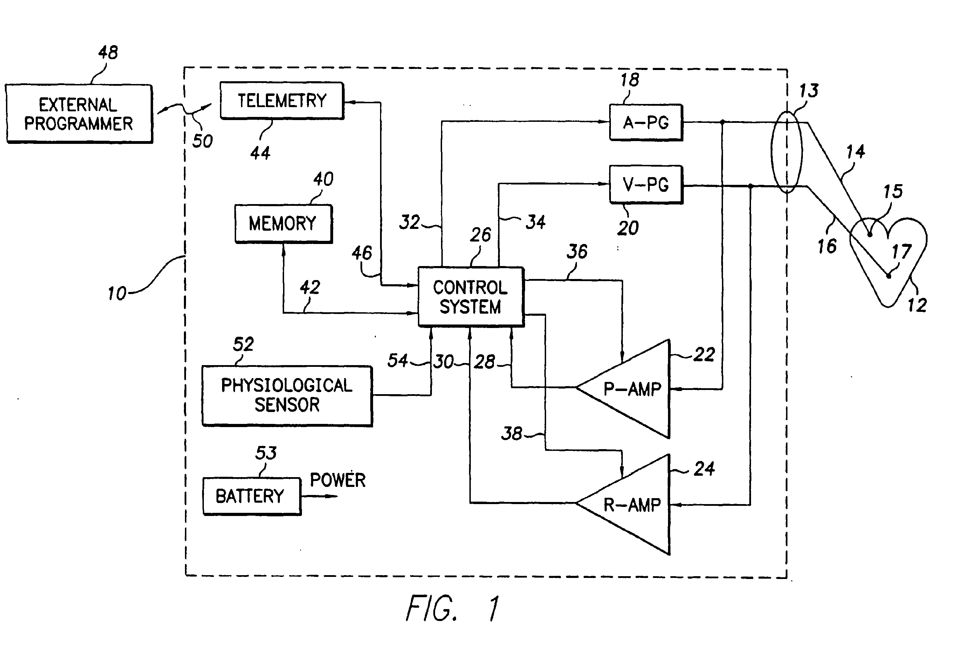

Referring first to **FIG. 1**, a simplified block diagram of the circuitry needed for a dual-chamber pacemaker **10** embodying the present invention is illustrated. The pacemaker **10** is coupled to a heart **12** by way of leads **14** and **16**, the lead **14** having an electrode **15** that is in contact with one of the atria of the heart, and the lead **16** having an electrode **17** that is in contact with one of the ventricles of the heart. The leads **14** and **16** are electrically and physically connected to the pacemaker **10** through a connector **13** that forms an integral part of the housing wherein the circuits of the pacemaker are housed.

The leads **14** and **16** carry stimulating pulses to the electrodes **15** and **17** from an atrial pulse generator **18** and a ventricular pulse generator **20,** respectively. Further, electrical signals from the atria are carried from the electrode **15** through the lead **14** to the input terminal of an atrial channel sense amplifier **22**; and electrical signals from the ventricles are carried from the electrode **17**, through the lead **16**, to the input terminal of a ventricular channel sense amplifier **24**.

The dual-chamber pacemaker **10** is controlled by a control system **26** that typically includes a microprocessor programmed to carry out control and timing functions. The control system **26** receives the output signals from the atrial amplifier **22** over a signal line **28**. Similarly, the control system **26** receives the output signals from the ventricular amplifier **24** over signal line **30.** The control system **26** also generates trigger signals that are sent to the atrial pulse generator **18** and the ventricular pulse generator **20** over signal lines **32** and **34,** respectively. These trigger signals are generated each time that a stimulation pulse is to be generated by the respective pulse generator **18** or **20.** The atrial trigger signal is referred to simply as the "A-trigger" and the ventricular trigger signal is referred to as the "V-trigger."

During the time that either an A-pulse or V-pulse is being delivered to the heart, the corresponding amplifier, **22** or **24**, is typically disabled by way of a blanking signal presented to these amplifiers from the control system or signal lines **36** and **38**, respectively. This blanking action prevents the amplifiers **22** and **24** from becoming saturated from the relatively large stimulation pulses that are present at their input terminals during this time. This blanking action also helps prevent residual electrical signals present in the muscle tissue as a result of the pacemaker stimulation from being interpreted as P-waves or R-waves.

As shown in **FIG. 1**, the pacemaker **10** further includes a memory circuit **40** that is coupled to the control system **26** over a suitable data/address bus **42.** This memory circuit **40** allows certain control parameters, used by the control system **26** in controlling the operation of the pacemaker, to be programmably stored and modified, as required, in order to customize the pacemaker's operation to suit the needs of a particular patient. For example, in accordance with the present invention, the control parameters and operating instructions required for a Wenckebach upper rate-response and the PMT detection of the present invention may be programmably stored in the memory **40.** Further, data sensed during the operation of the pacemaker may be stored in the memory **40** for later retrieval and analysis.

The memory **40** of the pacemaker **10** may take many forms, and may be subdivided into as many different memory blocks or sections (addresses) as needed in order to allow desired data and control information to be stored. For example, the memory **40** may store sensed data as a data record, which data record may then be used to guide the operation of the device. That is, the operating mode of the pacemaker may be dependent, at least in part, on past performance data. For example, an average atrial rate may be determined based on the sensed atrial rate over a prescribed period of time. This average rate may then be stored and updated at regular intervals. Such stored rate may then be compared to a preset atrial rate and, depending upon the difference, used to control the operating mode of the pacemaker. Other parameters, of course, in addition to or in lieu of atrial rate, may be similarly sensed, stored, averaged, or otherwise processed and then used for comparison purposes against one or more currently sensed parameters. Advantageously, modem memory devices allow for the storage of large amounts of data in this manner.

A telemetry/communications circuit **44** is connected to the control system **26** by way of a suitable command/data bus **46.** In tum, the telemetry circuit **44** which is included within the implantable pacemaker **10,** may be selectively coupled to an external programming device **48** by means of an appropriate communications link **50,** which communications line **50** may be any suitable electromagnetic link, such as an RF (radio frequency) channel, a magnetic link, an inductive link, an optical link, and the like. Advantageously, through the external programmer **48** and the communication link **50,** desired commands may be sent to the control system **26**. Similarly, through this communication link **50** with the programmer **48**, data commands (either held within the control system **26,** as in a data latch, or stored within the memory **40**) may be remotely received from the programmer **48.** Similarly, data initially sensed through the leads **14** and **16**, and processed by the microprocessor control circuits **26**, or other data measured within or by the pacemaker **10**, may be stored or uploaded to the programmer **48.** In this manner, non-invasive communications can be established with the implanted pacemaker **10** from a remote, non-implanted location.

The pacemaker **10** additionally includes a battery **53.** The battery provides operating power to all of the circuits of the pacemaker **10**.

It is noted that the pacemaker **10** in **FIG.1** is referred to as a dual-chamber pacemaker because it interfaces with both the atria and the ventricles of the heart. Those portions of the pacemaker **10** that interface with the atria, for example, the lead **14,** the P-wave sense amplifier **22**, the A-pulse generator **18,** and corresponding portions of the control system **26,** are commonly referred to as the "atrial channel." Similarly, those portions of the pacemaker **10** that interface with the ventricles, for example, the lead **16**, the R-wave sense amplifier **24**, the V-pulse generator **20,** and the corresponding portions of the control system **26**, are commonly referred to as the "ventricular channel."

As needed for certain applications, the pacemaker **10** may further include at least one sensor **52** that is connected to the control system **26** of the pacemaker **10** over a suitable connection line **54**. While this sensor **52** is illustrated in **FIG.1** as being included within the pacemaker **10,** it is to be understood that the sensor may also be external to the pacemaker **10,** yet still be implanted within in or carried by the patient. The common type of sensor is an activity sensor, such as a piezoelectric crystal, that is mounted to the case of the pacemaker. Other types of sensors are also known, such as, sensors that sense the oxygen content of the blood, respiration rate, pH of blood, body motion, and the like. The type of sensor used is not critical to the present invention. Any sensor, or combination of sensors, capable of sensing a physiological or physical parameter relatable to the rate at which the heart should be beating *(i.e.,* relatable to the metabolic need of a patient), and/or relatable to whether a tachyarrhythmia is likely to soon occur, can be used. Such sensors are commonly used with "rate-responsive" pacemakers in order to adjust the rate (pacing cycle) of the pacemaker in a manner that tracks the physiological or metabolic needs of the patient.

The telemetry or communications circuit **44** may be of conventional design, such as is described in U.S. Patent No. 4,944,299, or as is otherwise known in the art. Similarly, the external programmer **48** may be of any suitable design known in the art, such as is described in U.S. Patent No. 4,809,697. Likewise, the memory circuit **40,** and the circuits utilized in the atrial and ventricular channels may all be of common design as is known in the pacing art. The present invention is not concerned with the details of the circuitry illustrated for each of these pacing elements. Rather, it is concerned with the manner in which all of these pacing elements cooperate with each other in order to provide a particular pacing mode of operation. Such cooperation is controlled is by the control system **26**.

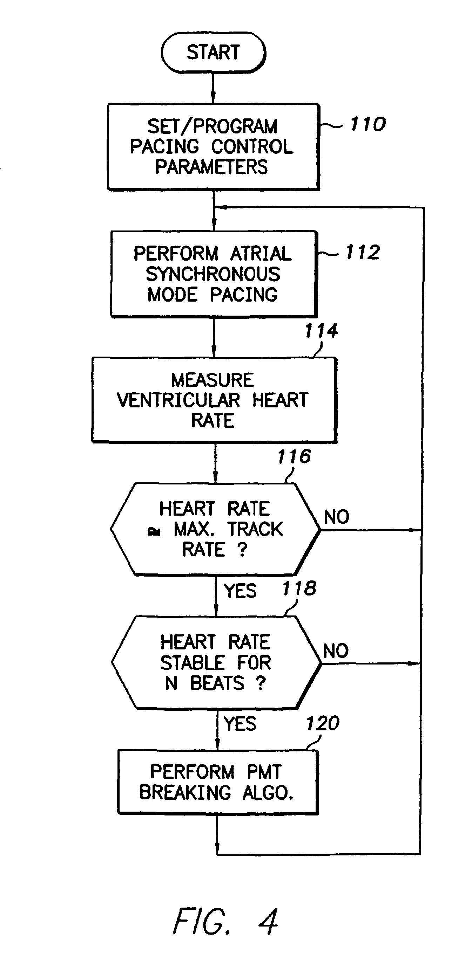

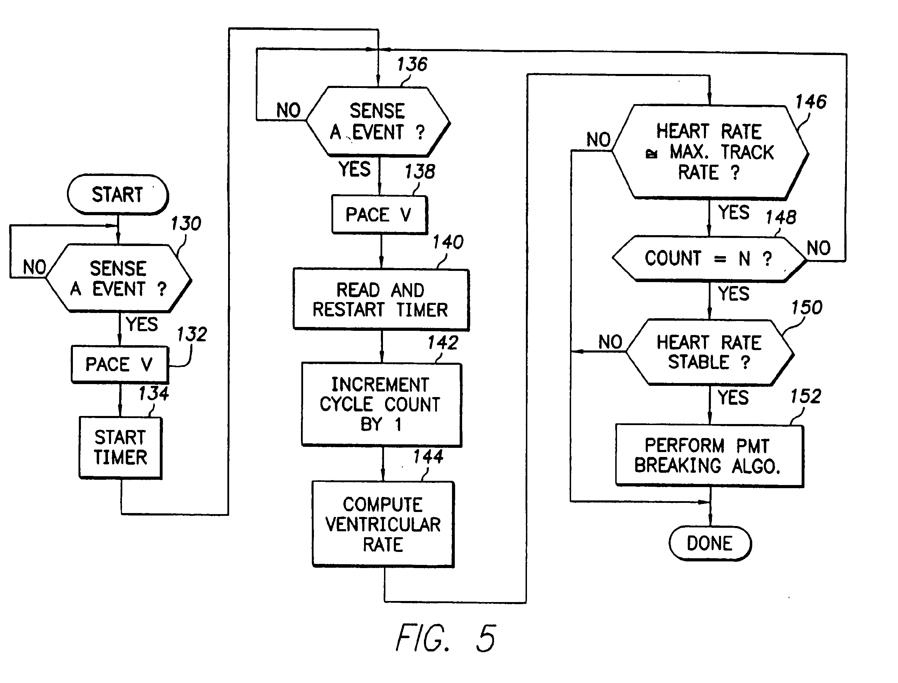

The control system **26** may be realized using a variety of different techniques and/or circuits. The preferred type of control system **26** is a microprocessor-based control system. It is noted, however, that the control system **26** could also be realized using a state machine. Indeed, any type of control circuit or system could be employed for the control system **26.** The present invention is likewise not concerned with the details of the control system **26.** Rather, it is concerned with the end result achieved by the control system. That is, so long as the control system **26** controls the operation of the pacemaker so that the desired functions are achieved as set forth herein, for example, by the following steps described below in the flowcharts of **FIGS. 4** **and** **5**, it matters little what type of control system is used. Those of skill in the implantable medical device art, given the teaches presented herein, should thus be able to fashion numerous different types of control systems or circuits that achieve the desired device control.

Representative of the types of control system that may be used with the invention is the microprocessor-based control system described in U.S. Patent No. 4,940,052, entitled "MICROPROCESSOR CONTROLLED RATE-RESPONSIVE PACEMAKER HAVING AUTOMATIC RATE RESPONSE THRESHOLD ADJUSTMENT." Reference is also made to U.S. Patent Nos. 4,712,555 and 4,944,298, wherein a state machine type of operation for a pacemaker is described; and U.S. Patent No. 4,788,980, wherein the various timing intervals used within the pacemaker and their inter-relationship are more thoroughly described.

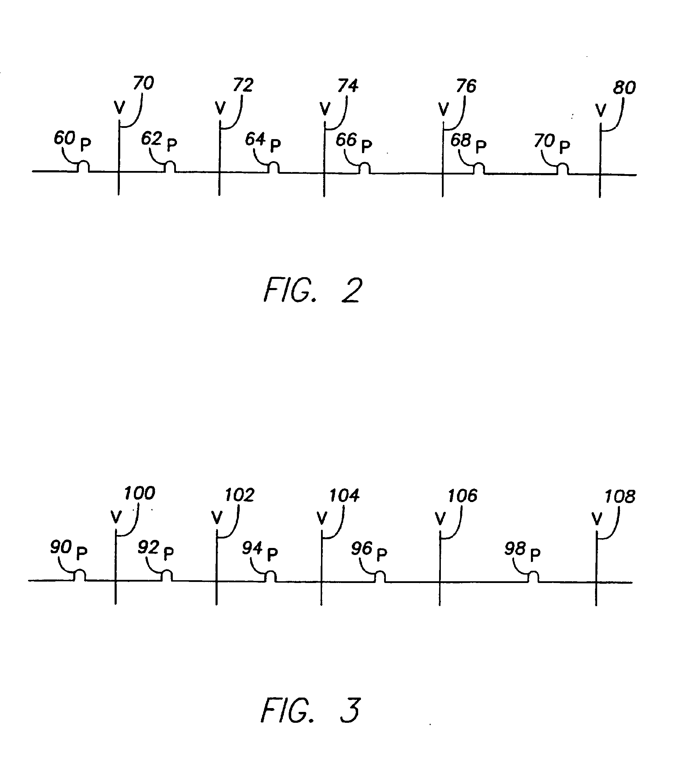

Referring now to the timing diagram of **FIG. 2****,** it illustrates the Wenckebach response which the pacemaker **10** of **FIG. 1** is programmed to provide when the intrinsic atrial rate rises above the preset or programmed upper rate limit of the pacemaker **10** during a sinus tachycardia. The P-waves **60, 62, 64, 66, 68** and **70** are occurring at a rate above the maximum tracking rate while the V-pulses **70, 72, 74,** and **76** are delivered by the ventricular pulse generator **20** at the maximum tracking rate. Because the P-waves are occurring at a rate faster than a maximum tracking rate, the PV interval of successive cardiac cycles is increasing and variably stretched. P-waves **60, 62, 64,** and **66** are tracked by the issuance of V-pulses **70, 72, 74,** and **76** respectively. However, P-wave **68** occurs soon enough after V-pulse **76** that it falls within the post-ventricular atrial refractory period (PVARP) of the pacemaker. The pacemaker therefore does not react to nor track P-wave **68.** The next P-wave **70** occurs outside of the PVARP and is thereafter tracked by the issuance of V-pulse **80.** Since the maximum tracking rate interval following V-pulse **76** has expired, the V-pulse **80** is delivered at the end of the programmed AV delay following P-wave **70.**

The foregoing illustrates a classic Wenckebach response employed in many modem day dual-chamber pacemakers. It will be noted that during the response, the PV intervals are not constant and are variably stretched. Also, and of equal importance, is the fact that the time between V-pulses **76** and **80** creates a pause or instability in the cardiac rate within just a few cardiac cycles.

Referring now to **FIG. 3**, it illustrates a timing diagram of a pacemaker mediated tachycardia at the maximum tracking rate. Retrograde conducted events sensed as P-waves **90, 92, 94, 96,** and **98** are occurring at the maximum tracking rate as are the V-pulses **100,102, 104, 106,** and **108.** The antegrade conduction (PV) intervals and retrograde conduction (VP) intervals remain constant. As a result, the cardiac rate remains stable at the maximum tracking rate.

In accordance with the present invention, the foregoing difference between the Wenckebach response and PMT cardiac event timing at the maximum tracking rate may be utilized to advantage in detecting PMTs at the maximum tracking rate. Further, the PMT detection at the maximum tracking rate may be accomplished within just a few cardiac cycles, and more specifically, within a number of cardiac cycles sufficient to evoke a Wenckebach response. This is in sharp contrast to the number of cardiac cycles required to detect a PMT by prior art methods.

Referring now to **FIG. 4**, it illustrates a flow diagram of the steps which the control system **26** of **FIG. 1** may take in detecting a PMT at the maximum tracking rate in accordance with the present invention. The process begins at step **110** wherein pacing control parameters are set by establishing the pacemaker in an atrial synchronous mode such as the DDD mode and programming other pacing parameters in a manner well known in the art. Following step **110,** the process moves to step **112** wherein atrial synchronized mode pacing is begun. After each cardiac cycle, step **114** is performed wherein the ventricular heart rate is measured. After each measurement of the ventricular heart rate, the control system **26** then determines in step **116** if the heart rate is at the maximum tracking rate. If the heart rate is not at the maximum tracking rate, the process returns to step **112**. If the heart rate is at the maximum tracking rate, the control system **26** then determines in step **118** if the heart rate has been stable over the last n number of beats. The number of beats (n) required is a number of beats which are sufficient for a Wenckebach response at the upper rate to be evoked. In accordance with this embodiment, the number of cardiac cycles required may be, for example, between 3 and 6.

The heart rate stability in accordance with step **118** may be performed by employing any one of a number of numerical analysis methods known in the art. For example, the control system **26** may maintain a standard deviation of the heart rate over the last n beats or, given the abrupt change in the heart rate during the Wenckebach response, it may compare the current heart rate with a heart rate determined during a previous cardiac cycle.

If in step **118** it is determined that the heart rate is not stable, this will indicate a Wenckebach response to a sinus tachycardia and the process will return to step **112**. However, if the heart rate is stable over the last n beats, the process then moves to step **120** wherein the control system performs a PMT breaking therapy or algorithm to break the PMT. PMT breaking therapies are well known in the art.

**FIG. 5** illustrates a flow diagram of the steps which may be employed by the control system **26** for detecting a PMT at the maximum tracking rate in accordance with further aspects of the present invention. The process illustrated in **FIG. 5** may be triggered by a determination that the heart rate is at the maximum tracking rate. The process illustrated in **FIG. 5****,** begins at step **130** wherein the control system **26** determines if an atrial event has been sensed by the atrial sense amplifier **24**. When an atrial event is sensed, the process moves to step **132** wherein a V-pulse is issued by the ventricular pulse generator **20.** The V-pulse is issued either after the preset or programmed AV delay or at the maximum tracking rate if the AV delay is completed prior to expiration of the maximum tracking rate interval. Following step **132**, the control system **26** then in step **134** starts a timer for determining the ventricular heart rate of the next cardiac cycle.

After starting the timer in step **134,** the process moves to step **136** wherein the control system determines if another atrial event has been sensed. When another atrial event is sensed, the processor then moves to step **138** for delivering another V-pulse. Again, the V-pulse is delivered either after the preset or programmed AV delay or at the end of the maximum tracking rate interval if the maximum tracking rate interval expires after the expiration of the AV delay. After the V-pulse is delivered in accordance with step **138,** the control system **26** then, in step **140,** reads and restarts the timer to begin timing the next cardiac interval.

In step **142,** the control system **26** increments a cycle counter by one count. The cycle counter is used to count up to the number of cardiac cycles (n) sufficient to evoke a Wenckebach response. The process then moves to step **144** wherein the ventricular rate is computed for the just completed cardiac cycle. Once the ventricular rate is computed, the process moves to step **146** to determine if the heart rate is still at the maximum tracking rate. If the heart rate is not at the maximum tracking rate, the process is complete. However, if the heart rate is still at the maximum tracking rate, then the control system **26** moves to step **148** to determine if the counter has reached n counts. If the counter has not reached n number of counts, the process then will return to step **136** to repeat the foregoing process for the next cardiac cycle. However, if the counter has counted n number of cardiac cycles, it then proceeds to step **150** to determine if the heart rate has been stable over the last n number of cardiac cycles. If the heart rate has not been stable, the process is complete inasmuch as it is now been determined that a Wenckebach response has been evoked resulting from a sinus tachycardia. However, if the heart rate is determined to have been stable, the process then moves to step **152** for performing the PMT breaking therapy or algorithm. When the PMT therapy is completed, the process is complete.

As can be seen from the foregoing, the present invention provides a pacemaker mediated tachycardia detection system and method for use in an implantable pacemaker having a Wenckebach upper rate-response. By discerning the difference between cardiac event timing between the Wenckebach response and a pacemaker mediated tachycardia, the system and method of the present invention is capable of detecting a PMT at the maximum tracking rate within a relatively few number of cardiac cycles. Hence, the system and method of the present invention is capable of responding faster to pacemaker mediated tachycardias at the maximum tracking rate as compared to existing PMT detection methods. In addition, false PMT detection is reduced thereby reducing pauses in pacemaker therapy.

While the invention has been described by means of specific embodiments and applications thereof, it is understood that one of skill in the art could readily adapt the PMT detection system by suitable microprocessor programming for determining cardiac rate by measuring the time spans between P-waves of the heart sensed by the atrial sense amplifier 22.

## Claims

1. An implantable pacemaker (10) having a Wenckebach upper rate-responses comprising: first means for sensing cardiac events (15,22) in an atrium of a heart (12); second means for delivering ventricular pacing pulses (17,20) to a ventricle of the heart (12); second means for delivering ventricular pacing pulses (17, 20) to a ventricle of the heart (12) during a number of cardiac cycles sufficient to evoke a Wenckebach response, wherein each ventricular pacing pulse is delivered following a sensed cardiac event in the atrium; and third means (20) for determining a cardiac rate during the number of cardiac cycles; **characterised by** a system for detecting a pacemaker mediated tachycardia at a maximum tracking rate comprising fourth means (20) for determining if the cardiac rate is substantially constant during the number of cardiac cycles wherein a substantially constant cardiac rate during the number of cardiac cycles is indicative of a pacemaker mediated tachycardia at the maximum tracking rate.

2. The pacemaker of Claim 1, further including fifth means (20) for determining if the cardiac rate is at the maximum tracking rate and wherein the fourth means (20) is responsive to the fifth means (20) determining that the cardiac rate is at the maximum tracking rate.

3. The pacemaker of Claim 1, wherein the third means (20) includes timing means for timing time spans between immediately successive ventricular pacing pulses for determining the cardiac rate.

4. The pacemaker of Claim 1, wherein the third means (20) includes timing means for timing time spans between immediately successive sensed cardiac events in the atrium for developing the cardiac rate.

5. The pacemaker of Claim 1, wherein the number of cardiac cycles is between 3 and 6.

6. The pacemaker of Claim 1, further including counting means for counting the number of cardiac cycles and wherein the fourth means (20) is responsive to the counting means counting the number of cardiac cycles for determining if the cardiac rate is substantially constant.

## Patentansprüche

1. Implantierbarer Herzschrittmacher (10) mit Wenckebach-Periodik Grenzfrequenzantworten, umfassend: ein erstes Mittel zum Erfassen von kardialen Ereignissen (15, 22) in einem Herzvorhof (12); ein zweites Mittel zum Liefern von ventrikulären Schrittmacherimpulsen (17, 20) an einen Herzvorhof (12); ein zweites Mittel zum Liefern von ventrikulären Schrittmacherimpulsen (17. 20) an einen Herzvorhof (12) in mehreren Herzzyklen, bis eine Antwort auf die Wenckebach-Periodik ausgelöst wird, wobei jeder ventrikuläre Schrittmacherimpuls nach einem erfassten kardialen Ereignis in dem Herzvorhof geliefert wird; und ein drittes Mittel (20) zum Bestimmen einer Herzfrequenz in den mehreren Herzzyklen;
**gekennzeichnet durch** ein System zum Detektieren einer schrittmacherinduzierten Tachykardie an einer maximalen Grenzfrequenz, umfassend ein viertes Mittel (20) zum Bestimmen, ob die Herzfrequenz in den mehreren Herzzyklen im Wesentlichen konstant ist, wobei eine in den mehreren Herzzyklen im Wesentlichen konstante Herzfrequenz indikativ für eine schrittmacherinduzierte Tachykardie an einer maximalen Grenzfrequenz ist.

2. Herzschrittmacher nach Anspruch 1,
ferner umfassend ein fünftes Mittel (20) zum Bestimmen, ob die Herzfrequenz bei einer maximalen Grenzfrequenz liegt, wobei das vierte Mittel (20) responsiv gegenüber dem fünften Mittel (20) bestimmt, dass die Herzfrequenz an der maximalen Grenzfrequenz liegt.

3. Herzschrittmacher nach Anspruch 1,
wobei das dritte Mittel (20) Mittel zum Takten der Zeitintervalle zwischen unmittelbar aufeinanderfolgenden ventrikulären Schrittmacherimpulsen zum Bestimmen der Herzfrequenz umfasst.

4. Herzschrittmacher nach Anspruch 1,
wobei das dritte Mittel (20) Mittel zum Takten der Zeitintervalle zwischen unmittelbar aufeinanderfolgenden erfassten kardialen Ereignissen in dem Herzvorhof zum Erstellen der Herzfrequenz umfasst.

5. Herzschrittmacher nach Anspruch 1,
wobei die Anzahl der Herzzyklen zwischen 3 und 6 liegt.

6. Herzschrittmacher nach Anspruch 1,
ferner umfassend Zählmittel zum Zählen der Anzahl der Herzzyklen, wobei das vierte Mittel (20) responsiv gegenüber dem Zählmittel zum Zählen der Anzahl der Herzzyklen bestimmt, ob die Herzfrequenz im Wesentlichen konstant ist.

## Revendications

1. Stimulateur cardiaque implantable (10) présentant une réponse de rythme supérieur de Wenckebach comprenant : des premiers moyens destinés à détecter des événements cardiaques (15, 22) dans une oreillette d'un coeur (12) ; des deuxièmes moyens destinés à délivrer des impulsions de stimulation ventriculaire (17, 20) à un ventricule du coeur (12) ; des deuxièmes moyens destinés à délivrer des impulsions de stimulation ventriculaire (17, 20) à un ventricule du coeur (12) pendant un certain nombre de cycles cardiaques suffisant pour évoquer une réponse de Wenckebach, dans lequel chaque impulsion de stimulation ventriculaire est délivrée à la suite d'un événement cardiaque détecté dans l'oreillette ; et des troisièmes moyens (20) destinés à déterminer un rythme cardiaque pendant le nombre de cycles cardiaques ; **caractérisé par** un système destiné à détecter une tachycardie induite par un stimulateur cardiaque à une fréquence de poursuite maximale comprenant des quatrièmes moyens (20) destinés à déterminer si le rythme cardiaque est sensiblement constant pendant le nombre de cycles cardiaques dans lequel un rythme cardiaque sensiblement constant pendant le nombre de cycles cardiaques indique une tachycardie induite par le stimulateur cardiaque à la fréquence de poursuite maximale.

2. Stimulateur cardiaque selon la revendication 1, comportant en outre des cinquièmes moyens (20) destinés à déterminer si le rythme cardiaque se situe à la fréquence de poursuite maximale et dans lequel les quatrièmes moyens (20) répondent aux cinquièmes moyens (20) déterminant que le rythme cardiaque se situe à la fréquence de poursuite maximale.

3. Stimulateur cardiaque selon la revendication 1, dans lequel les troisièmes moyens (20) comportent des moyens de minutage destinés à minuter les laps de temps entre des impulsions de stimulation ventriculaire immédiatement successives pour déterminer le rythme cardiaque.

4. Stimulateur cardiaque selon la revendication 1, dans lequel les troisièmes moyens (20) comportent des moyens de minutage destinés à minuter les laps de temps entre des événements cardiaques détectés immédiatement successifs dans l'oreillette pour développer le rythme cardiaque.

5. Stimulateur cardiaque selon la revendication 1, dans lequel le nombre de cycles cardiaques est compris entre 3 et 6.

6. Stimulateur cardiaque selon la revendication 1, comportant en outre des moyens de comptage destinés à compter le nombre de cycles cardiaques et dans lequel les quatrièmes moyens (20) répondent aux moyens de comptage comptant le nombre de cycles cardiaques pour déterminer si le rythme cardiaque est sensiblement constant.
